# EUROPÄISCHE PATENTANMELDUNG

(11) **EP 2 338 449 A1**
(43) Veröffentlichungstag der Anmeldung: **29.06.2011**
(21) Anmeldenummer: 10192728.3
(22) Anmeldetag: 26.11.2010
(51) Int. Cl.: A61F 13/02

(54) **Absorptionskörper zur Auflage auf Wunden**

(30) Priorität: 30.11.2009 DE 202009015670 U
(71) Anmelder: McAirlaid's Vliesstoffe GmbH & Co. KG, 37339 Berlingerode (DE)
(72) Erfinder: Schmidt, Andreas, 37115, Duderstadt (DE)
(74) Vertreter: Christophersen, Ulrich Rudolf

(57) **Zusammenfassung**

Es wird ein Absorptionskörper zum Auflegen auf Wunden beansprucht, welcher ausgebildet ist als mehrlagig aufgebauter Schichtkörper aus mindestens
A) einer der Haut zugewandten Lage A,
B) einer der Haut abgewandten Lage B,
C) einem zwischen der Lage A und der Lage B angeordneten Saugkern.

Der Absorptionskörper zeichnet sich dadurch aus, dass die Lage A im Wundbereich (W) ein für Flüssigkeiten durchlässiges Material aufweist und außerhalb des Wundbereichs zumindest teilweise mit einem Adhäsionsmittel versehen ist.

## Beschreibung

Die Erfindung betrifft einen Absorptionskörper zum Auflegen auf Wunden nach dem Oberbegriff des Patentanspruchs 1.

Absorptionskörper zum Aufnehmen von Körperflüssigkeiten finden nicht nur in der Hygiene sondern auch in der Medizin Anwendung, insbesondere zur Behandlung von Wunden.

Absorptionskörper, die als Wundauflagen eingesetzt werden, werden in der Regel auf die äußere Wunde gelegt, um das Eindringen von Fremdkörpern in die Wunde zu verhindern und Blut und Wundsekret aufzunehmen.

In der EP 1 507 498 B1 ist ein Absorptionskörper zum Auflegen auf Wunden, insbesondere zum Aufsaugen von Wundflüssigkeiten, offenbart. Der Absorptionskörper weist einen flachen Materialabschnitt aus Absorptionsmaterial, nämlich einem aufsaugenden Vlies mit darin verteilten Super-Absorberteilchen, sowie eine flüssigkeitsdurchlässige Hülle auf. Die Hülle umgibt den Materialabschnitt aus Absorptionsmaterial und bildet eine Barriere gegen feste Körperausscheidungen und sie verhindert den Durchtritt von anderen, ausgetretenen Substanzen zu dem innerhalb der Hülle angeordneten Absorptionsmaterial. Der Materialabschnitt aus Absorptionsmaterial weist eine Fläche auf, welche kleiner ist als die Fläche der Umhüllung, nämlich in seinem nicht benetzten Zustand 3 % bis 75 % der flachgelegten Hülle. Die Fläche der flachgelegten Hülle ist ferner durch eine Naht und ggf. durch eine Faltkante begrenzt. Die Hülle und/oder das Absorptionsmaterial können an ihrem/seinem Umfang mit einer an dem Körper des Patienten haftenden Substanz, wie Klebstoff, versehen sein. Als geeigneter Klebstoff werden Pektin-Zellulose-Verbindungen genannt.

Die aus dem Stand der Technik bekannten Wundauflagen haben den Nachteil, dass sie auf der Wunde mit einem entsprechenden Verband fixiert werden müssen. Auch verrutschen die Wundauflagen leicht, wenn ein Verband angelegt wird und beim späteren Tragen des Verbandes. Es fehlt an der Möglichkeit, die Wundauflage direkt auf der Haut des Patienten fixieren zu können.

Der Erfindung liegt demgemäß die Aufgabe zugrunde, einen Absorptionskörper zum Auflegen auf Wunden zur Verfügung zu stellen, der volle Aufsaugkapazität aufweist, jedoch auch ohne weitere Hilfsmittel, wie Verbandmaterial, auf der Wunde bzw. auf der Haut des Patienten fixiert werden kann.

Gegenstand der vorliegenden Erfindung ist ein Absorptionskörper der eingangs beschriebenen Art, der sich **dadurch auszeichnet, dass** die der Haut zugewandte Lage A im Wundbereich ein für Flüssigkeiten durchlässiges Material aufweist und außerhalb des Wundbereichs zumindest teilweise mit einem Adhäsionsmittel versehen ist.

Der erfindungsgemäße Absorptionskörper eignet sich insbesondere zum Auflegen auf Wunden, d. h. auf sogenannte äußere Wunden, die im Allgemeinen auf der Haut einschließlich der Schleimhaut des Patienten auftreten können. Bei diesen Wunden kann es sich um akute Verletzungen oder auch um chronische Wunden, also nicht-heilende Wunden, bzw. nur durch sekundäre Wundheilung heilende Wunden handeln.

Der erfindungsgemäße Absorptionskörper hat den Vorteil, dass die Wunde durch eine die austretende Körperflüssigkeit aufnehmende Lage bedeckt wird, wobei diese Lage in einem Bereich außerhalb des Wundbereichs zumindest teilweise mit einem Klebstoff als Adhäsionsmittel versehen ist. Dadurch ist es möglich, den Absorptionskörper auf der Körperoberfläche zu fixieren, ohne zusätzliches Verbandmaterial etc. zu Hilfe nehmen zu müssen. Das Fixieren des Absorptionskörpers ohne Verbandmaterial hat weiterhin den Vorteil, dass der Absorptionskörper auf einfache Weise auch vom Patienten selbst auf die Wunde aufgelegt und zum Haften gebracht werden kann. Sollte der Absorptionskörper zusätzlich mit einem Verband o. ä. fixiert oder abgedeckt werden, wird ein Verrutschen während des Verbindens und auch während des Tragens verhindert.

Der Absorptionskörper ist ein mehrlagig aufgebauter Schichtkörper, der im Wesentlichen aus einer der Haut zugewandten Lage A, einer der Haut abgewandten Lage B und einem Saugkörper, der zwischen diesen beiden Lagen angeordnet ist, besteht.

Die der Haut zugewandte Lage A liegt unmittelbar auf der Wunde und teils auf der die Wunde umgebenden Hautoberfläche auf. Um eine optimale Wundversorgung zu gewährleisten, besteht die Lage A in dem Bereich, in welchem sie unmittelbar auf der Wunde aufliegt, aus einem flüssigkeitsdurchlässigem Material. Dieses flüssigkeitsdurchlässige Material kann ausgewählt sein aus Materialien wie Nonwoven oder Gewebe und/oder gelochter zwei- oder dreidimensionaler Folie, die vorzugsweise nicht mit Wunden verkleben. Nonwoven-Materialien sind textil-ähnliche Materialien, die aus langen Fasern hergestellt sind und die durch chemische, mechanische, Hitze- oder Lösungsbehandlung miteinander verbunden sind. Es handelt sich hierbei um textile Flächengebilde aus einzelnen Fasern, die flüssigkeitsdurchlässig sind. Neben den Nonwoven (= Vliesstoffen) können auch gelochte oder strukturierte zwei- oder dreidimensionale Folien als Lage A eingesetzt werden. Die gelochten Folien weisen in ihrer zweidimensionalen Ausgestaltung in der Fläche Löcher auf, die einen Flüssigkeitsdurchlass des Materials gewährleisten. Gelochte dreidimensionale Folien zeichnen sich dadurch aus, dass durch die Lochung das gelochte Material sich über die Ebene des Flächengebildes erstreckt, wodurch eine dreidimensionale Struktur entsteht. Durch die dreidimensionale Struktur verringert sich die Auflagefläche auf der Wunde, was die Wundheilung weiter positiv beeinflussen kann und ein Verkleben von getrockneten Exsudat und Pflaster verhindert. Die Materialien, aus welchen die Nonwoven/Vliese bzw. die gelochten Folien hergestellt sein können, sind Polyolefin-Folien, wie Polyethylen- oder Polypropylen-Folien oder auch Naturstoffvliese.

Wesentlich ist, dass die Lage A im Bereich außerhalb der Wunde / des Absorptionskerns zumindest partiell mit einem Adhäsionsmittel und vorzugsweise Klebstoff versehen ist. Der Bereich der Wunde und vorzugsweise auch unmittelbar um die Wunde herum sollte jedoch frei von Klebstoff sein. In einer möglichen Ausgestaltung kann die Lage A aus dem bereits beschriebenen Vlies und/oder einer Folie bestehen, welche an seinen Rändern mit dauerhaft klebendem Klebstoff beaufschlagt, z. B. beschichtet, ist.

In einer weiteren Ausgestaltung kann die Lage A auch aus mehreren Zonen bzw. aus unterschiedlichen Materialien in unterschiedlichen Zonen bestehen, wobei nur Zonen außerhalb der Wunde / des Absorptionskerns, die auf der gesunden Hautoberfläche aufliegen, mit dem Adhäsionsmittel beaufschlagt sind. Das Adhäsionsmittel kann über den gesamten Umfang oder nur partiell, punktuell oder in Form von Streifen etc. aufgebracht sein. Menge des aufgebrachten Adhäsionsmittel und die Art und Weise, wie es aufgebracht wird, hängen in der Regel davon ab, ob der erfindungsgemäße Absorptionskörper auf der Wunde fixiert werden soll, um das Anbringen eines Verbandes zu erleichtern oder das Fixieren des Absorptionskörpers unterhalb des Verbandes zu unterstützen oder aber ob der Absorptionskörper während des Tragens ohne zusätzlichen Verband fixiert sein soll.

Als Klebstoffe kommen alle denkbaren hautverträglichen Klebstoffe in Betracht wie sie bei der Herstellung von Pflastern üblich und bekannt sind. Vorzugsweise sollte der Klebstoff ein dauerhaft klebender Klebstoff sein, der auch nach einmaliger Verwendung, d. h. wenn er bereits auf die Hautoberfläche aufgebracht und wieder abgezogen wurde, wieder verwendbar ist. So ist es möglich, die Position der Auflage noch einmal zu korrigieren. Auch sollte der Klebstoff vorzugsweise nach In-Kontakt-Kommen mit Wasser seine Klebkraft nicht verlieren. Ein bevorzugt eingesetzter Klebstoff ist sogenanntes untervernetztes Polyurethan, d. h. ein Polyurethan mit niedriger Kennzahl, eingesetzt werden. Untervernetzte Polyurethane werden durch Umsetzung von Polyisocyanaten mit langkettigen Polyolen, die vorzugsweise frei von kurzkettigen Anteilen sein sollen, hergestellt.

Die Oberseite des erfindungsgemäßen Absorptionskörpers, d. h. die der Haut abgewandten Seite, wird von der Lage B gebildet. Diese weist vorzugsweise die gleichen Abmessungen wie die Lage A auf. Um die Lagen A und B miteinander verbinden zu können, können sie, je nach Material, aus welchem diese Lagen gebildet sind, miteinander verklebt, verschweißt, gesiegelt oder in sonstiger Weise verbunden sein.

In einer weiteren Ausgestaltung sind die Maße einer der beiden Lagen A oder B größer als die der anderen Lage, der überstehende Rand kann so um den Rand der jeweils kleineren Lage umgeschlagen werden, wodurch die Außenränder geschlossen werden können.

Eine optimale Wundversorgung ist insbesondere dann möglich, wenn auch die der Haut abgewandte Lage eine Lage aus atmungsaktiven Materialien ist.

Die Materialien, aus welchen die Lagen A und B hergestellt sind, können gleich oder verschieden sein. Vorzugsweise ist die Lage B atmungsaktiv, d.h. sie ist für Wasserdampf durchlässig, nicht aber für Flüssigkeiten. Die Lage B kann beispielsweise eine atmungsaktive Folie sein, wie eine gelochte zwei- und/oder dreidimensionale Folie, eine atmungsaktives SMS, Nonwoven (Vliese) aus natürlichen oder synthetischen Fasern, und/oder ein Laminat aus unterschiedlichen Materialien, wie aus Nonwoven und atmungsaktiver Folie (BTBS-Filme =breathable film textile backsheet), wie sie üblicherweise für die Herstellung von Außenseiten von Inkontinenzprodukten bekannt sind.

Als dritte Lage weist der erfindungsgemäße Absorptionskörper einen Saugkern auf. Dieser ist von den Lagen A und B vorzugsweise nach Art einer Hülle umgeben. Der Saugkern dient dazu, die aus der Wunde ausgetretenen Exsudate aufzunehmen und dauerhaft zu absorbieren. Eine Rücknässung in Richtung zur Wunde sollte vorzugsweise weitgehend vermieden werden. Der Saugkern wird daher vorzugsweise von einem Material gebildet, welches dazu in der Lage ist, Körperflüssigkeiten nicht nur zu absorbieren, sondern auch zu speichern, wie Zellstoff oder Zellstoff-verwandte Materialien sowie synthetische saugfähige Materialien. Der Saugkern ist vorzugsweise ein Vlies aus Zellstofffasern, wie ein Airlaid.

Um die Absorptionsfähigkeit des Saugkerns weiter zu steigern, kann dieser Teilchen aus superabsorbierenden Polymeren enthalten. Diese können beispielsweise direkt bei der Herstellung des Saugkerns mit eingearbeitet werden.

In einer Ausführungsform ist der Saugkörper aus einer Faserstoffbahn aus Zellstoff-Fasern oder einer im Luftstrom aufgeschichteten Schüttung von Zellstofffasern (Fluff Pulp) hergestellt, die durch einen aus zwei Prägewalzen bestehenden Kalander geführt werden. Unter Erzeugung eines Prägemusters im Druckbereich werden die Fasern bindemittelfrei, punkt- oder linienförmig kalandriert und so verbunden. Als besonders geeignet hat sich ein Saugkern erwiesen, der gemäß dem in dem europäischen Patent 1 032 342 B1 beschriebenen Verfahren hergestellt wird.

Ist der Saugkörper C von den Lagen A und B nach Art einer Hülle umgeben, kann der Saugkörper an einer oder an beiden der Lagen A und B fixiert sein, oder er kann nur lose von den Lagen A und B umgeben sein. Um ein Verrutschen des Saugkörpers innerhalb dieser Hülle möglichst zu vermeiden und sicherzustellen, dass der Saugkörper zumindest vollständig oder teilweise die Lage A an der Stelle bedeckt, an welcher diese auf der Wunde aufliegt, sollte der Saugkörper mindestens die gleiche Größe haben wie die Wunde. In einer bevorzugten Ausführungsform ist der Saugkörper etwas kleiner als die Lage A.

Die Lagen A und B sind vorzugsweise an ihren Außenkanten verschlossen. In einer möglichen Ausgestaltung der vorliegenden Erfindung sind die Lagen A und B gleich bzw. nahezu gleich und die Kanten sind miteinander verklebt, verschweißt oder versiegelt. Diese Verbindung der beiden Lagen kann unmittelbar an den Kanten der Lagen erfolgen oder aber in einem gewissen Abstand von der Kante, so dass sich quasi ein umlaufender Saum bildet. Auch eine Verklebung bis zum Saugkern hin ist denkbar.

In einer weiteren Ausgestaltung der Erfindung ist die Fläche einer der beiden Lagen A und B größer als die andere, die überstehenden Flächen der größeren Lage können um die Kanten der kleineren Lage umgeschlagen werden, wodurch ebenfalls eine den Saugkörper umschließende Hülle entsteht.

Die Erfindung wird anhand der beigefügten Figuren näher erläutert. Es zeigen:
Fig. 1 eine Ansicht auf einen flächig gestalteten Absorptionskörper
Fig. 2 eine Ausführungsform des Absorptionskörpers in einer Schnittdarstellung
Fign. 3 - 7 weitere Ausführungsformen in Schnittdarstellung.

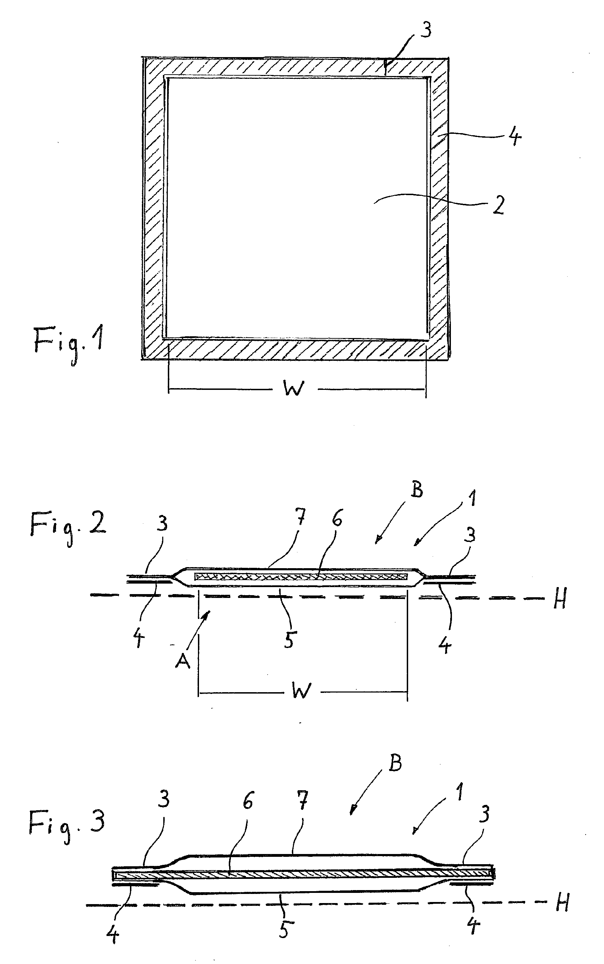

Fig. 1 zeigt eine Ansicht auf einen Absorptionskörper 1, und zwar mit Blickrichtung auf die bei der Anwendung der Haut zugewandte Seite. Diese erste Seite des Absorptionskörpers wird im Folgenden auch als Lage A bezeichnet.

Die der Haut zugewandte Lage 2 ist in ihren äußeren Bereichen 3, also außerhalb des Wundbereichs W, mit einem Klebstoff 4 beaufschlagt. Der Klebstoff 4 sollte möglichst nur in solchen Bereichen angeordnet oder aufgebracht sein, die, anders als die zentral angeordnete Lage 2, nicht mit der Wunde selbst in Berührung kommen. Um die Absorptionsfähigkeit des erfindungsgemäßen Absorptionskörpers nicht zu beeinträchtigen, sollten die Flächen der der Haut zugewandten Lage 2 möglichst frei von Beschichtungen etc. sein, so dass die Flüssigkeitsdurchlässigkeit dieser Lage erhalten bleibt. Darüber hinaus besteht die Gefahr, dass, wenn Klebstoff in die Nähe der Wunde gelangt, der Heilungsprozess beeinträchtigt werden kann.

In Fig. 2 ist eine erste Ausführungsform des erfindungsgemäßen Absorptionskörpers in einem schematischen Schnitt dargestellt. Der Absorptionskörper ist ein mehrlagig aufgebauter Schichtkörper 1 mit einer der Haut H zugewandten Lage 2, die entlang ihrer Außenbereiche 3 mit Klebstoff 4 beaufschlagt ist. Zwischen den mit Klebstoff 4 beaufschlagten Bereichen 3 befindet sich die Wundauflagefläche 5. Die Wundauflagefläche 5 ist flüssigkeitsdurchlässig, um Exsudate etc., die von der Wunde abgegeben werden, durchzulassen. Diese Exsudate werden vom zentralen Saugkörper 6 aufgenommen und darin gespeichert.

Auf der der Haut H abgewandten, im Folgenden auch als Lage B bezeichneten Seite weist der Absorptionskörper eine Schicht 7 auf, die die außenliegende Deckschicht des Absorptionskörpers darstellt. Die Deckschicht 7 besteht vorzugsweise aus atmungsaktiven Materialien, d. h. aus solchen Materialien, die für Dampf durchlässig sind, aber flüssigkeitsundurchlässig. Durch diese der Haut abgewandte Deckschicht sollte möglichst vermieden werde, dass Exsudat aus dem Absorptionskörper austritt und z. B. Kleidung verschmutzt.

In der in Fig. 2 dargestellten Ausführungsform sind die der Haut H zugewandte Lage 2 und die der Haut abgewandte Lage 7 entlang eines Randbereichs 3 miteinander verbunden, der in der hier dargestellten Ausführungsform eine umlaufende Zone bildet. Die Verbindung der Lagen in dieser Zone kann punktförmig sein, so dass die Schichten 2 und 7 in den Außenbereichen 3 nicht miteinander verbunden sind. Sie können auch vollflächig miteinander verbunden sein. In dem Bereich 5 liegt der Absorptionskörper auf der Haut H auf, dieser Bereich erstreckt sich über die gesamte Wunde und vorzugsweise auch über den Bereich der Haut, der unmittelbar dem Wundbereich benachbart ist und ggf. darüber hinaus. So kann vermieden werden, dass die Wunde mit dem Adhäsionsmittel 4 in Kontakt kommt.

In der in Fig. 3 dargestellten Ausführungsform erstreckt sich der Saugkörper bis nahe der Außenkanten 3, wo er beim Verbinden der Lagen bzw. Schichten 2 und 7 miterfasst wird und so zwischen diesen Lagen fixiert ist.

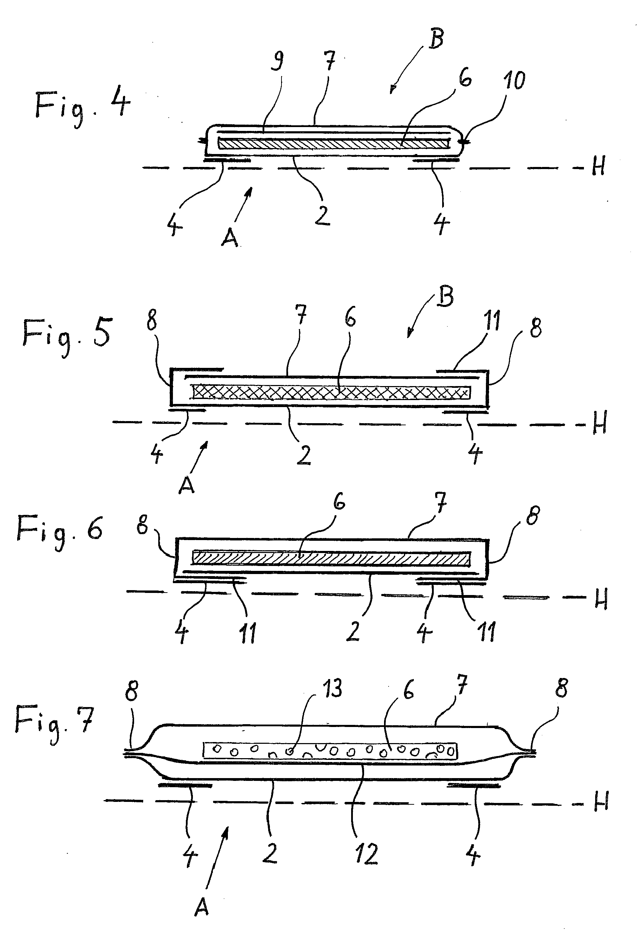

In Fig. 4 ist eine weitere Ausführungsform des Absorptionskörpers dargestellt. In dieser Ausführungsform ist zwischen dem Saugkörper 6 und der der Haut H abgewandten Lage 7 eine weitere Lage bzw. Schicht 9 angeordnet. Diese Schicht kann aus einem feinen Vlies oder Tissue bestehen. Sie soll verhindern, dass, wenn der Absorptionskörper bereits sehr lange auf der Wunde aufliegt und die Absorptionsfähigkeit erschöpft ist, Flüssigkeit nach außen dringt.

In der in Fig. 4 dargestellten Ausführungsform sind die Lagen 2 und 7 durch nur partielles Verschweißen oder eine Punktverschweißung miteinander verbunden. Es bilden sich lokale Verbindungen 10 zwischen den Lagen 2 und 7, ohne dass eine weitere umlaufende Kante auftritt.

In den Fign. 5 und 6 sind Ausführungsformen dargestellt, bei denen die Lagen 2 und 7 unterschiedliche Größen haben. In der in Fig. 5 dargestellten Ausführungsform ist die Fläche der der Haut H zugewandten Lage größer als die der Haut abgewandten Lage, so dass die Überstände 11 umgeschlagen werden können und auf der Lage 7 fixiert werden, wodurch sich geschlossene Außenkanten 8 bilden.

Fig. 6 zeigt eine Ausführungsform, in welcher die der Haut H abgewandte Schicht 7 größer ist als die der Haut zugewandte Schicht 2. Daher kann die Schicht 8 umgeschlagen werden und einen geschlossenen Rand um die Schicht 2 und um den Saugkörper 6 bilden.

Fig. 7 zeigt eine weitere Ausführungsform des erfindungsgemäßen Absorptionskörpers. In dieser Ausführungsform ist zwischen dem Saugkörper 6 und der der Haut H zugewandten Lage 2 eine weitere Lage oder Schicht 12 angeordnet. Diese ist vorzugsweise ein dünnes Vlies oder ein Tissue, das flüssigkeitsdurchlässig für die aufzunehmenden Körperflüssigkeiten und Exsudate ist. Die Lage 12 kann zum einen die Aufgabe haben, zu verhindern, dass im Saugkörper angeordnete Superabsorberteilchen 13 in Richtung Wunde austreten können. Zum anderen verleiht die Lage 12 dem Saugkörper 6 eine gewisse Festigkeit, für den Fall, dass die Absorptionskapazität des Saugkörpers 6 gesättigt ist und durch weitere Aufnahme von Flüssigkeit die Gefahr besteht, dass der Saugkern zu stark aufquillt und ggf. zerfällt.

### Bezugszeichenliste

- 1: Absorptionskörper
- 2: (der Haut zugewandte) Lage oder Schicht
- 3: äußerer Bereich
- 4: Adhäsionsmittel, Klebstoff
- 5: Wundauflagefläche
- 6: Saugkörper, Saugkern
- 7: (der Haut abgewandte) Lage oder Schicht
- 8: Kantenbereich
- 9: weitere Lage oder Schicht
- 10: lokale Verbindung
- 11: Überstand
- 12: weitere Lage
- 13: Superabsorber

- H: Haut
- W: Wundbereich

## Patentansprüche

1. Absorptionskörper zum Auflegen auf Wunden, ausgebildet als mehrlagig aufgebauter Schichtkörper aus mindestens
A) einer der Haut zugewandten Lage A,
B) einer der Haut abgewandten Lage B,
C) einem zwischen der Lage A und der Lage B angeordneten Saugkern, **dadurch gekennzeichnet, dass** die Lage A im Wundbereich (W) ein für Flüssigkeiten durchlässiges Material aufweist und außerhalb des Wundbereichs zumindest teilweise mit einem Adhäsionsmittel versehen ist.

2. Absorptionskörper nach Anspruch 1, **dadurch gekennzeichnet, dass** das für Flüssigkeiten durchlässige Material ausgewählt ist aus Nonwoven, Gewebe und/oder gelochter zwei- oder dreidimensionaler Folie.

3. Absorptionskörper nach Anspruch 1 oder 2, **dadurch gekennzeichnet, dass** das Adhäsionsmittel ein dauerhaft klebender Klebstoff ist.

4. Absorptionskörper nach Anspruch 3, **dadurch gekennzeichnet, dass** der Klebstoff untervernetztes Polyurethan enthält.

5. Absorptionskörper nach einem der Ansprüche 1 bis 4, **dadurch gekennzeichnet, dass** die Lage B aus atmungsaktivem Material besteht.

6. Absorptionskörper nach einem der Ansprüche 1 bis 5, **dadurch gekennzeichnet, dass** der Saugkern ausgewählt ist aus Zellstoff oder Zellstoff-verwandten Materialien.

7. Absorptionskörper nach Anspruch 6, **dadurch gekennzeichnet, dass** der Saugkern A aus einer Faserstoffbahn aus Zellstoff-Fasern oder einer im Luftstrom aufgeschichteten Schüttung von Zellstoff-Fasern hergestellt, die durch einen aus zwei Prägewalzen bestehenden Kalander geführt werden.

8. Absorptionskörper nach einem der Ansprüche 1 bis 7, **dadurch gekennzeichnet, dass** der Saugkern Teilchen aus superabsorbierenden Polymeren enthält.

9. Absorptionskörper nach Anspruch 5, **dadurch gekennzeichnet, dass** das atmungsaktive Material ausgewählt ist aus gelochten Folien, atmungsaktiven SMS-Folien, Nonwoven aus natürlichen oder synthetischen Fasern, Laminaten aus Nonwoven und atmungsaktiven Folien und/oder BTBS-Folien.

10. Absorptionskörper nach einem der Ansprüche 1 bis 9, **dadurch gekennzeichnet, dass** die Lagen A und B den Saugkern C umhüllen, und dass die Ränder der Lagen A und B geschlossen sind.

11. Absorptionskörper nach einem der Ansprüche 1 bis 10, **dadurch gekennzeichnet, dass** der Saugkern ein Flächengebilde ist mit Abmessungen, die kleiner sind als die Fläche der Lagen A bzw. B.
